(19)

Europäisches
Patentamt
European
Patent Office
Office européen
des brevets

(11) **EP 1 638 988 B1**

(12) **EUROPEAN PATENT SPECIFICATION**

(45) Date of publication and mention
of the grant of the patent:
**31.03.2010 Bulletin 2010/13**

(21) Application number: **04730316.9**

(22) Date of filing: **29.04.2004**

(51) Int Cl.:
*C07J 41/00* $^{(2006.01)}$   *C07J 1/00* $^{(2006.01)}$

(86) International application number:
**PCT/HU2004/000031**

(87) International publication number:
**WO 2005/000868 (06.01.2005 Gazette 2005/01)**

(54) **PROCESS FOR THE SYNTHESIS OF HIGH PURITY D-(17ALPHA)-13-ETHYL-17HYDROXY-18,19-DINORPRE:GN-4-ENE-20-YNE-3-ONE-OXIME**

VERFAHREN ZUR HERSTELLUNG VON HOCHREINEM D-(17ALPHA)-13-ETHYL-17-HYDROXY-18,19-DINOR-PREGN-4-ENE-20-YNE-3-ONE-OXIM

PROCEDE DE SYNTHESE D'UNE D-(17ALPHA)-13-ETHYL-17-HYDROXY-18,19-DINOR-PREGN-4-ENE-20-YNE-3-ONE-OXIME TRES PURE

(84) Designated Contracting States:
**AT BE BG CH CY CZ DE DK EE ES FI FR GB GR
HU IE IT LI LU MC NL PL PT RO SE SI SK TR**

(30) Priority: **30.06.2003 HU 0301982**

(43) Date of publication of application:
**29.03.2006 Bulletin 2006/13**

(73) Proprietor: **Richter Gedeon Nyrt.
1103 Budapest (HU)**

(72) Inventors:
• **TUBA, Zoltán
  H-1022 Budapest (HU)**
• **MAHO, Sándor
  H-1183 Budapest (HU)**
• **KISS, János
  H-1074 Budapest (HU)**
• **MAGYARI, Endréné
  H-2730 Albertirsa (HU)**

• **TERDY, László
  H-1192 Budapest (HU)**

(74) Representative: **HOFFMANN EITLE
Patent- und Rechtsanwälte
Arabellastrasse 4
81925 München (DE)**

(56) References cited:
**AT-B- 348 151      US-A- 4 027 019
US-A- 5 876 746**

• **SISENWINE, SAMUEL F. ET AL: "The conversion of d-norgestrel-3-oxime-17-acetate to d-norgestrel in female rhesus monkeys" CONTRACEPTION , 15(1), 25-37 CODEN: CCPTAY; ISSN: 0010-7824, 1977, XP008035682**
• **RUFER C ET AL: "[Total synthesis of optically active 13-ethyl-gonane derivatives]" JUSTUS LIEBIGS ANNALEN DER CHEMIE. 1967, vol. 702, 1967, pages 141-148, XP002297402 ISSN: 0075-4617**

EP 1 638 988 B1

## Description

The invention relates to a process for the synthesis of high purity d-(17α)-13-ethyl-17-hydroxy-18,19-dinorpregn-4-ene-20-yne-3-one-oxime (further on norelgestromine) via acetylation of d-norgestrel at position 17, oximation of the oxo group at position 3 of the obtained 17-acetoxy derivative, and finally hydrolyzing the acetoxy group at position 17 of the obtained 3-oxime derivative.

The term "high purity" - as used in this specification - means products/materials/compounds, in which the content of the specified compound is at least 99.5 mass percent and the overall amount of other steroid impurities is not more, than 0.1 mass percent.

The investigation of the metabolism of norgestimate is described in the following publication: Am.J.Obstet Gynecol. 163, 2127-31. (1990). The authors discovered, that after oral application the metabolites of d-norgestimate are the norelgestromine, d-(17α)-13-ethyl-17-acetoxy-18,19-dinorpregn-4-ene-20-yne-3-one (norgestrel acetate), as well as the d-(17α)-13-ethyl-17-hydroxy-18,19-dinorpregn-4-ene-20-yne-3-one (d-norgestrel), which are first of all responsible for the biological activity.

The efficacy and safety of norgestimate/ethynyl-oestradiol used as third generation contraceptive are described in the following publication: Am.J.Obstet Gynecol., 166, 1969-77. (1992). They also determined, that the main metabolite of norgestimate is norelgestromine, which has a similar pharmacological profile, than norgestimate and after oral administration it is detectable in the blood serum already after a short period of time.

In the U.S. patent Number of 5,876,746 the authors suggested the use of norelgestromine as such or in combination with an oestrogen component in transdermal plaster for inhibition of fertility.

In the Hungarian patent Number of 165356 the synthesis of dl- as well as d-norgestrel is disclosed. The starting material of the synthesis is the racemic or the optically active 3-methoxy-gona-2,5(10)-diene-177i-ol, which is reacted with hydroxylammonium chloride in pyridine at 100 ˚C, then the obtained 13-ethyl-[3-(hydroxy-imino)]-gon-4-ene-17β-ol is oxidized at position 17, followed by ethynylation of the oxo group at position 17 to give the dl-(17α)-13-ethyl-17-hydroxy-18,19-dinorpregn-4-ene-20-yne-3-one-oxime, or norelgestromine. Although the synthesis of the intermediates, the dl-(17α)-13-ethyl-17-hydroxy-18,19-dinorpregn-4-ene-20-yne-3-one-oxime and norelgestromine, are described in recipes, the identification and the purity (the quality requirements) as well as the biological activity of these compounds are not given in the patent. Contraception Vol. 15(1) pp 25-37, 1977 also discloses the synthesis of norgestimate.

In the U.S. patent Number of 4,027,019 mainly the synthesis of the 17-acetoxy and the ester derivatives of norelgestromine in general, as well as the biological examinations thereof are described.

Considering, that to meet the more and more demanding requirements of pharmacopoeia is a basic requirement for every active ingredient applied in therapy, especially for steroids having high biological activity, we made an effort to elaborate an economical process for producing such high purity product, which meets even the most demanding requirements as well.

The purity of the desired compound is also determined by the purity of the starting materials. This is especially important, if the purification of the final product can be carried out only with large loss of material, because in this case the economical realization of the process can be a limit of the accessible purity. However in many cases providing the sufficiently pure starting material can also be carried out with large loss of material. In this case, it can be tried to take advantage of the more advantageous physical property of the last or any of the previous intermediates for producing the pure final product.

Surprisingly it was found, that an order of magnitude more pure, than the purity limit (maximum amount of impurity is less than 1 %) usually given in pharmacopoeias, norelgestromine can be prepared according to our invention as follows:

the starting material, d-(17α)-17-hydroxy-13-ethyl-18,19-dinorpregn-4-ene-20-yne-3-one (d-norgestrel) - purity of a least 93%, preferably 93-94 % - is acetylated with acetic anhydride in acetic acid, in the presence of zinc chloride and hydrogen chloride, or 70 % perchloric acid in an inert gas atmosphere, and after completion of the reaction the excess of acetic anhydride and the "enol acetate" by-product are decomposed with aqueous hydrochloric acid, then the formed d-(17α)-17-acetoxy-13-ethyl-18,19-dinorpregn-4-ene-20-yne-3-one is isolated from the reaction mixture by addition of ice-water, the precipitated product is filtered off, washed with water, dried, dissolved in dichloromethane or acetone and clarified with silica gel or aluminum oxide and charcoal, after filtration of the clarifier the resulted solution is concentrated and the residue is recrystallized, preferably from a 9:1 mixture of diisopropyl ether/acetonitrile or diisopropyl ether/ethanol, the obtained d-(17α)-17-acetoxy-13-ethyl-18,19-dinorpregn-4-ene-20-yne-3-one is reacted either with hydroxylammonium acetate or with hydroxylammonium chloride in the presence of sodium acetate, in acetic acid in nitrogen atmosphere under vigorous stirring for about 1 hour, after completion of the reaction water is added, the precipitated product is filtered off, washed with water, dried and recrystallized preferably from ethanol, the obtained d-(17α)-17-acetoxy-13-ethyl-18,19-dinorpregn-4-ene-20-yne-3-one-oxime is hydrolyzed with an equivalent amount of an alkali metal hydrox-

ide in a $C_1$-$C_4$ alkanol solution, in nitrogen atmosphere between a temperature of about 5-38 ˚C, under vigorous stirring, after completion of the reaction the mixture is diluted with water and the pH of the resulted suspension is adjusted to 7,5-9 with acetic acid, the precipitated product is filtered off, washed with water, dried, the crude product is dissolved in ethanol, clarified with charcoal, and after filtration of the clarifier water is added to the obtained solution, the precipitated high purity d-(17α)-17-hydroxy-13-ethyl-18,19-dinorpregn-4-ene-20-yne-3-one-oxime is filtered off, washed with water and optionally recrystallized from ethanol.

The above mentioned "enol acetate" is a labile compound having a 3-acetoxy-3,5-diene structure. This structure is formed in small amounts as product of an equilibrium by-reaction under the circumstances of the first acetylation step and is decomposed under forming d-(17α)-17-acetoxy-13-ethyl-18,19-dinorpregn-4-ene-20-yne-3-one with aqueous hydrochloric acid.

According to our invention it is also possible, that the geometrical isomers are separated from the anti/syn isomeric mixture formed in the oximation step before the hydrolysis by known methods, for example by chromatography, and the so obtained compounds are hydrolyzed. Therefor, as the hydrolysis according to our invention does not influence the geometry of the nitrogen atom of the oxime, after the hydrolysis the appropriate, pure anti and syn isomers of norelgestromine are obtained.

The invention is illustrated by the following not limiting examples.

## Example 1

### d-(17α)-13-Ethyl-17-acetoxy-18,19-dinorpren-4-ene-20-yne-3-one (Norgestrel acetate)

Under nitrogen, to a vigorously stirred suspension of 150 g (about 0.45 mol) of d-norgestrel (purity 94 %) and 1500 ml of acetic acid 135 ml (1.428 mol) of acetic anhydride and 3 ml of 70 % aqueous perchloric acid are added. The suspension becomes clear in a few minutes. Stirring is continued for 20 min, then 135 ml of water and 75 ml of 10 % aqueous hydrochloric acid is added to the reaction mixture. After stirring for 1 h the reaction mixture is poured into 14 1 of ice-water. The precipitated product is filtered off, washed with water and dried. The obtained crude product is dissolved in 1500 ml of dichloromethane and stirred with 150 g of silica gel for 30 min for clarifying. The silica gel is filtered off and the solvent is evaporated. The residue is refluxed with a 9:1 mixture of isopropyl ether/acetonitrile for 15 min, then the solution is cooled to 0 ˚C. the precipitated product is filtered off and dried to yield 137 g of the pure title compound. A further 19 g of the title compound can be obtained from the mother liquor by repeating the above purification process. Overall yield: 152 g (89.3 %). Mp.: 204-205 ˚C. $[\alpha]_D$= -25˚ (c=1% chloroform)

According to thin layer chromatography the product contains less than 1 % of impurity (calculated for levonorgestrel acetate). (For TLC 25 DC-Alufolien Kieselgel 60 $F_{254}$ plates and a 4:1 mixture of toluene-acetone, as eluent were used. Detection was carried out by spraying the plates with a mixture of ethanol-sulfuric acid.)

## Example 2

### d-(17α)-13-Ethyl-17-acetoxy-18,19-dinorpregn-4-ene-20-yne-3-one (Norgestrel acetate)

Under nitrogen, to a stirred suspension of 10 g (about 0.03 mol) of d-norgestrel (purity 93 %) and 100 ml of acetic acid 6 ml (0.063 mol) of acetic anhydride, 2 g of anhydrous zinc chloride and 1.6 ml of 6.7 % hydrogen chloride solution in acetic acid are added. The suspension becomes clear in a few minutes. Stirring is continued for 20 min, then 5 ml of water and after a further 15 min of stirring 3 ml of 18 % aqueous hydrochloric acid are added to the reaction mixture, which is stirred for a further 45 min. Then the reaction mixture is poured into 600 ml of ice-water, the precipitated product is filtered off, washed with water and dried. The crude title compound is purified according to the method described in example 1.

Yield: 15.4 g (90.47 %). Mp.: 204-205 ˚C. $[\alpha]_D$= -25˚ (c=1 % chloroform). Maximum impurity is 1 % according to the analysis described in example 1.

## Example 3

### d-(17α)-13-Ethyl-17-acetoxy-18,19-dinorpregn-4-ene-20-yne-3-one-oxime (Norgestimate)

Under nitrogen, to a stirred solution of 12 g (0.033 mol) of d-norgestrel acetate, obtained according to example 1 or 2, and 120 ml of acetic acid 9.44 g (0.1 mol) of hydroxylammonium acetate is added. The reaction mixture is stirred at room temperature for 45 min, then it is poured into 11 of water. The precipitated crystals are filtered off, washed with water and dried below 40˚C in vacuum. The obtained 12.2 g of crude product is dissolved in 250 ml of boiling ethanol, clarified with 1.2 g of charcoal, and after filtration of charcoal the solution is concentrated to a volume of about 20 % of the original one. The so obtained solution containing the crystalline product is cooled to 0 ˚C and kept at this temperature for 12 h. the precipitated crystals are filtered off, washed with ethanol and dried below 40 ˚C to yield 11.0 g (88 %) of the title compound. Mp.: 224-226 ˚C.

According to Test 1 and Test 2 described in USP 26th Pharmacopoeia on page 1335 the impurity of the product is less, than 0.5 %.

## Example 4

### d-(17α)-13-Ethyl-17-acetoxy-18,19-dinorpregn-4-ene-20-yne-3-one-oxime (Norgestimate)

Under nitrogen, to a vigorously stirred solution of 120 g (0.33 mol) of norgestrel acetate, obtained according to examples 1 or 2, and 1259 g (1200 ml) of acetic acid 90.2 g (1.1 mol) of anhydrous sodium acetate and 76 g (1.93 mol) of hydroxylammonium hydrochloride are added. The temperature of the reaction mixture should be below 30 ˚C. The reaction is completed in 1 h. Then the obtained white suspension is poured into 10 l of water and the obtained mixture is stirred for 30 min. The precipitated product is filtered off, washed with water and dried at 40˚C.

The obtained crude product (122 g) is dissolved in 197.3 g (2500 ml) of boiling ethanol, clarified with 12 g of charcoal, filtered and the filtrate is concentrated under reduced pressure below 40 ˚C to a volume of 400 ml, then cooled to 0-5 ˚C and kept on this temperature for 3 h. The precipitated white crystalline product is filtered off, washed with 197 g (250 ml) of ethanol in two portions and dried below 40 ˚C to yield 102 g (81.6 %) of the title compound. Mp.: 224-226 ˚C.

According to Test 1 and Test 2 described in USP 26th Pharmacopoeia on page 1335 the impurity of the product is less, than 0.5 %.

## Example 5

### d-(17α)-13-Ethyl-17-hydroxy-18,19-dinorpregn-4-ene-20-yne-3-one-oxime (Norelgestromine)

Under nitrogen, to a stirred suspension of 50.0 g (135.3 mmol) of norgestimate, obtained according to example 3 or 4, and 500 ml of methanol 17.04 g (0.406 mol) of lithium hydroxide monohydrate is added at 20-28 ˚C. After stirring for about 30 min a homogeneous solution is obtained, and the temperature rises 10 ˚C. The reaction mixture is stirred at 25-35 ˚C for 3 h and the completion of the reaction is checked by thin layer chromatography. Then the reaction mixture is poured into 5 l of water at 10-25 ˚C (the pH of the suspension is about 13), and the pH of the suspension is adjusted to 7.5-9 with 14.7 ml (0.25 mol) of acetic acid. The so obtained suspension is stirred for 20 min, then the crystalline product is filtered off and washed with 2 x 200 ml of water. The pH of the filtrate is checked and washing is repeated until the pH of the filtrate is 7-7.5. The filtered crude product is dried at 50 ˚C. The obtained 45 g of crude product is dissolved in 440 ml of ethanol at 25-30 ˚C, then 2.2 g of charcoal is added. After 20 min stirring the clarifier is filtered off and washed with ethanol. Then the filtrate is poured into 4.4 l of water at 10-25 ˚C under vigorous stirring. The obtained product is filtered off, washed with water and dried at 50 ˚C to yield 42.0 g (94.8 %) of the title compound. Mp.: 110-130 ˚C.

Water content: 0.4 %.

## Example 6

### d-(17α)-13-Ethyl-17-hydroxy-18,19-dinorpregn-4-ene-20-vne-3-one-oxime (Norelgestromine)

Under nitrogen, to a stirred suspension of 10.0 g (0.027 mol) of norgestimate (levonorgestrel-acetate-oxime), obtained according to example 3 or 4, and 100 ml of methanol 3.25 g (0.081 mol) of sodium hydroxide is added at 22 ˚C. After stirring for about 10 min a homogeneous solution is obtained, and the temperature rises to 32 ˚C. Then the reaction mixture is stirred at 25 ˚C for 3 h and the completion of the reaction is checked by thin layer chromatography. The reaction mixture is poured into 1000 ml of water at 10-20 ˚C under stirring and the pH of the suspension is adjusted to 7-7.5 with 3 ml of acetic acid. Then the obtained suspension is stirred for 20 min, the precipitated product is filtered off, washed with water and dried in vacuum at 40 ˚C over phosphorous pentoxide to yield 8.4 g (94.8 %) of the title compound as a mixture of 3E and 3Z isomers. Mp.: 110-130 ˚C. According to HPLC the amount of all the impurities is 0.09 %.

## Example 7

### d-(17α)-13-Ethyl-17-hydroxy-18,19-dinorpregn-4-ene-20-yne-3-one-oxime (Norelgestromine)

Under nitrogen, to a stirred suspension of 10.0 g (0.027 mol) of norgestimate (levonorgestrel-acetate-oxime), obtained according to example 3 or 4, and 100 ml of methanol 4.56 g (0.081 mol) of potassium hydroxide is added at 22 ˚C. After stirring for about 10 min a homogeneous solution is obtained, and the temperature rises to 32 ˚C. Then the reaction mixture is stirred at 25 ˚C for 3 h and the completion of the reaction is checked by thin layer chromatography. The reaction mixture is poured into 1000 ml of water at 10-20 ˚C under stirring and the pH of the suspension is adjusted to 7-7.5 with 2.7 ml of acetic acid. Then the obtained suspension is stirred for 20 min, the precipitated product is filtered off, washed with water and dried in vacuum at 40 ˚C over phosphorous pentoxide to yield 8.6 g (96.9 %) of the title compound as a mixture of 3E and 3Z isomers. Mp.: 110-130 ˚C. According to HPLC the amount of all the impurities is 0.1 %.

The purity of the obtained norelgestromine samples was determined by HPLC using Shimadzu instrument, UV detection and Shimadzu integrator. Detection was performed at 244 nm. 150 x 4.6 mm column was used, filled with 5 μm size Supelcosil LC-18-DB. packing material. A 7:25:68 mixture of acetonitrile:tetrahydrofuran:water was used as eluent. The determination was carried out at room temperature, with a flow rate of 1 cm$^3$/min.

The sample solution was prepared as follows: 25 mg of the compound was measured into a 50 ml volumetric flask, 5 ml of methanol was added and the sample was dissolved, then the flask was filled with the eluent until the calibration line. The standard solution (STD) was prepared the same way from analytical purity norelgestromine, containing the E/Z isomers in a ratio between 1.3-1.6.

The amount of impurity in % (S%) was calculated by the following formula:

$$S\% = \frac{C_{STD} \times A_{impurity}}{A_{STDcalc} \times C_{sample}} \times r_f \times 100$$

In the formula:

| | |
|---|---|
| A = | area under the curve for the component in the subscript |
| $C_{STD}$ = | concentration of the standard solution [mg/ml] |
| $A_{STD\ calc.}$ = | $A_{STD,E}$ x 0.72 + $A_{STD,Z}$ (subscript E means the data of E isomer, sub-script Z means that of the Z isomer) |
| $C_{sample}$ = | concentration of the sample solution [mg/ml] |

The response factors ($r_f$) of the known components are as follows:

| | |
|---|---|
| levonorgestrel | = 0.78 |
| norelgestromine Z isomer | = 1.00 |
| norelgestromine E isomer | = 0.72 |
| norgestimate Z isomer | = 1.08 |
| norgestimate E isomer | = 0.81 |
| in the case of unknown impurity | = 1.00 |

The result of the above HPLC determination is altogether 0.1 % of impurities.

## NMR measurements:

$^1$H NMR {500MHz, DMSO-$d_6$(TMS), $\delta$(ppm) Z/E isomer: 0.92 / 0.92 (3H,t,-CH$_2$-CH$_3$), 1.40 / 1.40 (2H, m,-**CH$_2$**-CH$_3$), 2.05 & 2.24 / 1.87 & 2.87 (2H,m,H-2), 3.28 / 3.28 (1H,s,$\equiv$ CH), 5.23 / 5.23 (1H,s,17-OH), 6.40 / 5.78 (1H,m,H-4),10.12/10.38 (1H,s,=N-OH)
$^{13}$C NMR {125MHz, DMSO-$d_6$(TMS), $\delta$(ppm) Z/E isomer}: 9.4/9.4 (-CH$_2$-CH$_3$), 18.3/18.3 (-CH$_2$-CH$_3$), 26.9 / 20.6 (C-2), 79.6/79.6 (C-17), 89.1/89.1 (-C$\equiv$), 74.9 / 74.9 ($\equiv$CH), 111.6 / 118.6 (C-4), 151.2 / 154.3 (C-3), 152.0 / 148.1 (C-5)

## Claims

**1.** Process for the synthesis of high purity d-(17$\alpha$)-13-ethyl-17-hydroxy-18,19-dinorpregn-4-ene-20-yne-3-one-oxime via acetylation of d-norgestrel at position 17, oximation of the oxo group at position 3 of the obtained 17-acetoxy derivative, and finally hydrolyzing the acetoxy group at position 17 of the obtained 3-oxime derivative, **characterized by**
carrying out the acetylation of the starting material, d-(17$\alpha$)-17-hydroxy-13-ethyl-18,19-dinorpregn-4-ene-20-yne-3-one (d-norgestrel) - purity at least 93 % -, with acetic anhydride in acetic acid, in the presence of zinc chloride and hydrogen chloride, or 70 % perchloric acid in an inert gas atmosphere, and after completion of the reaction decomposing the excess of acetic anhydride and the "enol acetate" by-product with aqueous hydrochloric acid,
then isolating the formed d-(17$\alpha$)-17-acetoxy-13-ethyl-18,19-dinorpregn-4-ene-20-yne-3-one from the reaction mixture by addition of ice-water, filtering off the precipitated product, washing with water, drying, dissolving in dichloromethane or acetone and clarifying with silica gel or aluminum oxide and charcoal, concentrating the resulted solution after filtration of the clarifier and recrystallizing the residue,
reacting the obtained d-(17$\alpha$)-17-acetoxy-13-ethyl-18,19-dinorpregn-4-ene-20-yne-3-one either with hydroxylammonium acetate or with hydroxylammonium chloride in the presence of sodium acetate, in acetic acid in nitrogen atmosphere under vigorous stirring for about 1 hour, addition of water after completion of the reaction; filtering off the precipitated product, washing with water, drying and recrystallizing,
hydrolyzing the obtained d-(17$\alpha$)-17-acetoxy-13-ethyl-18,19-dinorpregn-4-ene-20-yne-3-one-oxime with an equivalent amount of an alkali metal hydroxide in a $C_1$-$C_4$ alkanol solution, in nitrogen atmosphere between a temperature of about 5-38 ˚C, under vigorous stirring, diluting the reaction mixture with water after completion of the reaction and adjusting the pH of the resulted suspension to 7,5-9 with acetic acid, filtering off the precipitated product, washing with water, drying, dissolving the crude product in ethanol, clarifying with charcoal, and addition of water after filtration of the clarifier to the obtained solution, filtering off the precipitated high purity d-(17$\alpha$)-17-hydroxy-13-ethyl-18,19-dinorpregn-4-ene-20-yne-3-one-oxime, washing with water and optionally recrystallizing from ethanol.

**2.** The process according to claim 1, **characterized by** hydrolyzing the d-(17$\alpha$)-17-acetoxy-13-ethyl-18,19-dinorpregn-4-ene-20-yne-3-one-oxime in methanol with lithium hydroxide monohydrate.

**3.** The process according to claim 1, **characterized by** recrystallizing the obtained d-(17α)-17-acetoxy-13-ethyl-18,19-dinorpregn-4-ene-20-yne-3-one from a 9:1 mixture of diisopropyl ether/acetonitrile or diisopropyl ether/ethanol.

4. The process according to claim 1, **characterized by** recrystallizing the obtained d-(17α)-17-hydroxy-13-ethyl-18,19-dinorpregn-4-ene-20-yne-3-one-oxime from ethanol.

### Patentansprüche

1. Verfahren zur Synthese von hochreinem d-(17α)-13-Ethyl-17-hydroxy-18,19-dinorpregn-4-en-20-in-3-on-oxim durch Acetylierung von d-Norgestrel an der 17-Position, Oximierung der Oxogruppe an der 3-Position des erhaltenen 17-Acetoxyderivats, und abschliessende Hydrolyse der Acetoxygruppe an der 17-Position des erhaltenen 3-Oximderivats, **gekennzeichnet durch**
Durchführen der Acetylierung des Ausgangsmaterials d-(17α)-17-Hydroxy-13-ethyl-18,19-dinorpregn-4-en-20-in-3-on (d-Norgestrel) - mit mindestens 93 %-iger Reinheit - mit Acetanhydrid in Essigsäure in Gegenwart von Zinkchlorid und Chlorwasserstoff oder 70 %-iger Perchlorsäure in einer Schutzgasatmosphäre und Zersetzung des Überschusses an Acetanhydrid und dem Enolacetat-Nebenprodukt mit wässriger Salzsäure nach Abschluss der Reaktion,
Isolieren des gebildeten d-(17α)-17-Acetoxy-13-ethyl-18,19-dinorpregn-4-en-20-in-3-ons aus der Reaktionsmischung **durch** Zugabe von Eiswasser, Abfiltrieren des ausgefallenen Produkts, Waschen mit Wasser, Trocknen, Lösen in Dichlormethan oder Aceton und Klären mit Kieselgel oder Aluminiumoxid und Holzkohle, Konzentrieren der erhaltenen Lösung nach Abfiltrieren des Klärungsmittels und Umkristallisieren des Rückstands,
Umsetzen des erhaltenen d-(17α)-17-Acetoxy-13-ethyl-18,19-dinorpregn-4-en-20-in-3-ons entweder mit Hydroxylammoniumacetat oder mit Hydroxylammoniumchlorid in Gegenwart von Natriumacetat in Essigsäure unter Stickstoffatmosphäre unter kräftigem Rühren für etwa 1 Stunde, Zugabe von Wasser nach Abschluss der Reaktion, Abfiltrieren des ausgefallenen Produkts, Waschen mit Wasser, Trocknen und Umkristallisieren,
Hydrolysieren des erhaltenen d-(17α)-17-Acetoxy-13-ethyl-18,19-dinorpregn-4-en-20-in-3-on-oxims mit einer äquivalenten Menge eines Alkalimetallhydroxids in einer $C_{1-4}$-Alkanollösung unter Stickstoffatmosphäre bei einer Temperatur von etwa 5 bis 38°C unter kräftigem Rühren, Verdünnen der Reaktionsmischung mit Wasser nach Abschluss der Reaktion und Einstellen des pH-Werts der resultierenden Suspension auf 7,5 bis 9 mit Essigsäure, Abfiltrieren des ausgefallenen Produkts, Waschen mit Wasser, Trocknen, Lösen des Rohprodukts in Ethanol, Klären mit Holzkohle, Zugabe von Wasser nach Abfiltrieren des Klärungsmittels, Abfiltrieren des ausgefallenen, hochreinen d-(17α)-17-Hydroxy-13-ethyl-18,19-dinorpregn-4-en-20-in-3-on-oxims, Waschen mit Wasser und gegebenenfalls Umkristallisieren aus Ethanol.

2. Verfahren gemäss Anspruch 1, **dadurch gekennzeichnet, dass** das d-(17α)-17-Acetoxy-13-ethyl-18,19-dinorpregn-4-en-20-in-3-on-oxim mit Lithiumhydroxidmonohydrat in Methanol hydrolysiert wird.

3. Verfahren gemäss Anspruch 1, **dadurch gekennzeichnet, dass** das erhaltene d-(17α)-17-Acetoxy-13-ethyl-18,19-dinorpregn-4-en-20-in-3-on aus einer 9:1-Mischung von Diisopropylether/Acetonitril oder Diisopropylether/Ethanol umkristallisiert wird.

4. Verfahren gemäss Anspruch 1, **dadurch gekennzeichnet, dass** das erhaltene d-(17α)-17-Hydroxy-13-ethyl-18,19-dinorpregn-4-en-20-in-3-on-oxim aus Ethanol umkristallisiert wird.

### Revendications

1. Procédé de synthèse de d-(17α)-13-éthyl-17-hydroxy-18,19-dinorprégn-4-ène-20-yne-3-one-oxime de haute pureté par l'acétylation de d-norgestrel à la position 17, l'oximation du groupe oxo à la position 3 du dérivé 17-acétoxy obtenu, et enfin l'hydrolyse du groupe acétoxy à la position 17 du dérivé 3-oxime obtenu, **caractérisé par**
la réalisation de l'acétylation de la matière première, la d-(17α)-17-hydroxy-13-éthyl-18,19-dinorprégn-4-ène-20-yne-3-one (d-norgestrel) - de pureté d'au moins 93 % - , avec de l'anhydride acétique dans de l'acide acétique, en présence de chlorure de zinc et de chlorure d'hydrogène, ou de l'acide perchlorique à 70 % dans une atmosphère de gaz inerte, et après achèvement de la réaction en décomposant l'excès d'anhydride acétique et le sous-produit « énol acétate » avec de l'acide chlorhydrique aqueux,
puis l'isolement de la d-(17α)-17-acétoxy-13-éthyl-18,19-dinorprégn-4-ène-20-yne-3-one du mélange de réaction par l'addition d'eau glacée, l'élimination par filtration du produit précipité, le lavage avec de l'eau, le séchage, la dissolution dans du dichlorométhane ou de l'acétone et la clarification avec du gel de silice ou de l'oxyde d'aluminium et du charbon, la concentration de la solution obtenue après filtration du clarificateur et la recristallisation du résidu, la réaction de la d-(17α)-17-acétoxy-13-éthyl-18,19-dinorprégn-4-ène-20-yne-3-one avec de l'acétate d'hydroxylammonium ou bien du chlorure d'hydroxylammonium en présence d'acétate de sodium, dans de l'acide acétique dans une atmosphère d'azote sous agitation vigoureuse pendant environ 1 heure, l'addition d'eau après achèvement de la réaction, l'élimination par filtration du produit précipité, le lavage avec de l'eau, le séchage et recristallisation, l'hydrolyse du d-(17$\alpha$)-17-acétoxy-13-éthyl-18,19-dinorprégn-4-ène-20-yne-3-one-oxime obtenu avec une quantité équivalente d'un hydroxyde de métal alcalin dans une solution d'alcanol en C$_1$ à C$_4$, dans une atmosphère d'azote à une température d'environ 5 à 38˚C, sous agitation vigoureuse, la dilution du mélange de réaction avec de l'eau après achèvement de la réaction et l'ajustement du pH de la suspension obtenu à 7,5 à 9 avec de l'acide acétique, l'élimination par filtration du produit précipité, le lavage avec de l'eau, le séchage, la dissolution du produit brut dans de l'éthanol, la clarification avec du charbon, et l'addition d'eau après filtration du clarificateur à la solution obtenue, l'élimination par filtration du d-(17$\alpha$)-17-hydroxy-13-éthyl-18,19-dinorprégn-4-ène-20-yne-3-one-oxime, de haute pureté précipité, le lavage avec de l'eau et éventuellement la recristallisation à partir d'éthanol.

2. Procédé selon la revendication 1, **caractérisé par** l'hydrolyse de la d-(17$\alpha$)-17-acétoxy-13-éthyl-18,19-dinorprégn-4-ène-20-yne-3-one-oxime dans du méthanol avec du monohydrate d'hydroxyde de lithium.

3. Procédé selon la revendication 1, **caractérisé par** la recristallisation du d-(17$\alpha$)-17-acétoxy-13-éthyl-18,19-dinorprégn-4-ène-20-yne-3-one-oxime obtenu à partir d'un mélange à 9 : 2 de diisopropyl éther/acétonitrile ou de diisopropyl éther/éthanol.

4. Procédé selon la revendication 1, **caractérisé par** la recristallisation du d-(17$\alpha$)-17-hydroxy-13-éthyl-18,19-dinorprégn-4-ène-20-yne-3-one-oxime obtenu à partir d'éthanol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• HU 165356

• US 4027019 A

**Non-patent literature cited in the description**

• *Am.J.Obstet Gynecol.,* 1990, vol. 163, 2127-31

• *Am.J.Obstet Gynecol.,* 1992, vol. 166, 1969-77
• *Contraception,* 1977, vol. 15 (1), 25-37